# EUROPEAN PATENT APPLICATION

(11) **EP 3 977 979 A1**
(43) Date of publication of application: **06.04.2022**
(21) Application number: 20000357.2
(22) Date of filing: 02.10.2020
(51) Int. Cl.: A61K 8/92, A61Q 19/06

(54) **NATURAL THERAPY FOR PREVENTION OF EARLY STRETCH MARK DEVELOPMENT**

(71) Applicant: Lalvani, Tej, London W1U 2NT (GB); Shelatkar, Rohit, London W9 3SP (GB); Taylor, Robert, London W9 1AH (GB)
(72) Inventor: Lalvani, Tej, London W1U 2NT (GB); Shelatkar, Rohit, London W9 3SP (GB); Taylor, Robert, London W9 1AH (GB)
(74) Representative: Snaith, James Michael

(57) **Abstract**

This invention concerns a cosmetic or pharmaceutical preparation intended for application to skin. The aim of the preparation is to have a preventive effect on the formation of stretch marks through a reduction in the contractile forces developed by fibroblasts during early stretch mark development, through a specific 100% natural combination of bio-active oils. The preparation does not include any paraffin oils, petroleum derivatives, additives or waxes. The preparation includes : Avocado Oil, Olive Oil, Coconut Oil, Wheatgerm Oil, Macadamia Nut Oil, Shea Butter, Natural Mixed Tocopherols, Argan Oil, Rose Hip Oil, Calendula Oil, Natural Orange Oil, Lavender Oil, and Vitamin A Palmitate.

## Description

### TECHNICAL FIELD

This invention concerns a cosmetic or pharmaceutical preparation intended for application to skin in order to prevent the formation of stretch marks using a combination of 100% bio-active oils.

### BACKGROUND ART

Stretch marks are narrow streaks or lines that occur on the surface of the skin. Medically, they are often referred to as stria, striae or, during pregnancy, striae gravidarum. Stretch marks are usually long and thin, and often red or purple to start with, before gradually fading to a silvery-white colour. Stretch marks can occur anywhere where the skin has been stretched, but they usually affect areas where fat is stored.

Stretch marks can occur : commonly during pregnancy, after rapid weight gain, in body builders and weight lifters, during puberty, when there is a family history of stretch marks, when there is health condition or a syndrome, such as Cushing's syndrome or Marfan syndrome, or after the prolonged or inappropriate use of corticosteroids. They affect around 70% of adolescent women and 40% of adolescent men.

Stretch marks often occur during the later stages of pregnancy, affecting about 8 out of 10 pregnant women. Whether or not stretch marks occur depends on the skin type and how elastic it is. During pregnancy, hormones are produced that soften the ligaments in the pelvis so that they are more flexible when giving birth. Ligaments are strong bands of tissue that connect joints. However, hormones also soften the fibres in the skin, making it prone to stretch marks. As the baby grows and the mother's skin stretches, she may get stretch marks on her abdomen. They might also develop on the thighs and breasts as they increase in size. Stretch marks usually fade and become less noticeable after childbirth, but they do not always disappear completely.

There are a wide variety of methods of treating stretch marks. However, they are often of limited effect.

Cosmetic camouflage (make-up) can be used for small areas of skin affected by stretch marks. Some types are waterproof and can last for two to three days. Creams, gels and lotions are aimed at removing stretch marks, but do not usually prevent stretch marks from occurring. These products are essentially skin moisturisers.

Laser therapy can not completely remove stretch marks, but it may help fade them and make them less noticeable. Several different types of laser therapy are used to treat stretch marks. Pulsed dye laser treatment is one type of laser treatment available. It is painless and can be used at an early stage, while the stretch marks are still red or purple.

Cosmetic surgery for stretch marks is expensive and so is rarely recommended.

### DISCLOSURE OF INVENTION

The aim of the preparation is to have a preventive effect on the formation of stretch marks through a reduction in the contractile forces developed by fibroblasts during early stretch mark development. The active formulation consists of a specific 100% natural combination of bio-active oils. The preparation does not, unlike other preparations, include any paraffin oils, petroleum derivatives, additives or waxes.

Stretch marks are cutaneous lesions occurring frequently in weight gain during puberty, pregnancy and early adult life. Fibroblast cells are responsible for the production of the extracellular matrix and collagen of connective tissues. One possible cause of stretchmarks is a reduction in fibroblast activity. Previous studies show that highest contractile forces were developed by fibroblasts from early stretch marks (SMF) compared to those developed by fibroblasts from normal healthy skin (HF) of the same patient,

The active ingredients of the formulation of the preparation are given as percentages of the total weight of the formulation. The active ingredients are:

| | |
|---|---|
| Avocado Oil | 30.0 % |
| Olive Oil | 25.0 % |
| Coconut Oil | 15.0 % |
| Wheatgerm Oil | 14.0 % |
| Macadamia Nut Oil | 10.172% |
| Shea Butter | 2.0% |
| Argan Oil | 1.0% |
| Calendula Oil | 1.0% |
| Rose Hip Oil, | 1.0% |
| Natural Mixed tocopherols | 0.648% |
| Orange Oil (5 Fold Ext.) | 0.096% |
| Lavender Oil | 0.072% |
| Vitamin A Palmitate | 0.0117% |
| Total : | 100% |

The invention is unique in the range of natural ingredients which have been shown to provide a preventive effect on the formation of stretch marks. demonstrated by quantification of contractile forces developed by fibroblasts from early stretch marks (red color) and from healthy surrounding skin using GlasBoxPlus device.

### CLINICAL ASSESSMENT

The effects of the invention were assessed on fibroblasts from early stretch marks (red colour) of a woman in order to avoid the appearance of late stretch marks (white colour). These obtained results were compared to a basic control lotion already sold. The effects of two actives on contractiles forces were also tested on healthy fibroblasts.

### MATERIALS

For the obtaining and amplification of cells, early stretch marks fibroblasts (SMF) and healthy fibroblasts (HF) were obtained from a young woman.

### METHODS

After thawing, the cells were grown in Dulbecco's modified Eagle's medium supplemented with 10% of foetal calf serum, 40 mg/l of gentamicin and 2 mg/l of fungizone (DMEMc), in an incubator at 37°C, 5% CO2 and 95% air. Culture medium was changed twice a week and cells were subcultured when confluence was reached, by trypsinization. Cells were used under 10 passages.

### EVALUATION OF CELL VIABILITY

The study of cell viability in the presence of the actives was carried out using a MTT test. MTT is a salt of tetrazolium whose cycle is transformed by mitochondrial dehydrogenases. The yellow substrate is transformed into a dark blue product by living cells, but not by dead cells or the culture medium. This colorimetric test makes it possible to relate the intensity of colour to the number of living cells: the absorbence at 550 nm is directly proportional to the number of living fibroblasts.

The invention was directly diluted in culture medium. Plates of 96 wells were seeded with 5000 fibroblasts per well. After 24 hours of adherence, actives were added. Eleven concentrations of each active were tested : C1 0.00001, C2 0.00005, C3 0.00010, C4 0.00050, C5 0.00100, C6 0.00500, C7 0.01000, C8 0.05000, C9 0.10000, C10 0.50000, C11 1.00000. After 24 hours of culture in the presence of the actives, MTT tests were performed.

### STUDY OF THE CONTRACTILE FORCES

The cell chamber is composed of eight rectangular culture wells in which lattices develop. Two opposite beams hang down into each well at a distance of 27 mm apart. The lattice is attached to this sensor through a grid etched on the lower part of each beam. The beams have a system to detect their own deformation, which is proportional to the contractiles forces developed by fibroblasts. The signal is collected by a computer, which includes a specific program to give directly the forces in real time.

Fibroblasts were resuspended just before confluence with trypsin-EDTA (1X) solution. A sample was taken to determine the number of cells obtained. The suspension was centrifuged and brought to the concentration of 8.105 cells/ml. Fibroblasts were embedded three-dimensionally in hydrated collagen gels using a modified version of the technique developed by Bell.

Briefly the mixture was composed of:
6 volumes of 1.76X (DMEMc, NaHCO3, NaOH, antibiotics)
+ 3 volumes murine type I collagen (2 mg/ml)
+ 1 volume of cellular suspension (8.105 cells/ml)

The lattice mixture was poured into the rectangular culture plate of the GlasBoxPlus and polymerized in less than 30 min at 37°C. Immediately after lattice formation, actives were added.

The GlasBoxPlus was then placed into a humidified incubator at 37°C, and forces measurements were started after 30 minutes of stabilization, for 24 hours. The forces were expressed as arbitrary units after 24 hours of measurement.

### CALCULATIONS AND STATISTICAL ANALYSIS

Each GlasBoxPlus curve was fitted with GraphPad Prism software to determine the area under the curve (AUC), the maximum of contraction (Max) and the slope (speed of contraction).

The area under the curve gives information on the global contraction of fibroblasts during all the experiment. Maximum of contraction corresponds with the plateau of the fitted curve.

Data was expressed as mean ± sem. A variance analysis with one factor was performed for the study of cytotoxicity, area under the curve, maximum of contraction and the slope and was followed if necessary by a Fisher test.

A variance analysis with two factors (group versus control and time) was carried out for the study of measurement of contractile forces followed if necessary by a Fisher test. A p value less than 0.05 was considered significant.

### EVALUATION OF CELL VIABILITY IN THE PRESENCE OF THE INVENTION

Viability of fibroblasts was significantly decreased for concentrations of 0.005% to 0.5% of the invention. The increase in cell viability observed with 1% of active was due to interference between the invention and culture medium. All the concentrations tested up to until 0.001% could be used for the experiments.

### One Way Analysis of Variance

Data source: Data 1 in Moyenne_Stats_FVR_Huile Normality Test: Failed (P < 0,050)
Equal Variance Test: Failed (P < 0,050)

| Group name | N | Missing | Mean | SD | SEM |
|---|---|---|---|---|---|
| FVR | 10 | 0 | 100,000 | 14,934 | 4,723 |
| C1 | 10 | 0 | 94,516 | 10,666 | 3,373 |
| C2 | 10 | 1 | 102,186 | 18,794 | 6,265 |
| C3 | 10 | 0 | 103,548 | 24,273 | 7,676 |
| C4 | 10 | 0 | 99,032 | 20,277 | 6,412 |
| C5 | 10 | 0 | 98,442 | 12,949 | 4,095 |
| C6 | 10 | 0 | 75,484 | 11,013 | 3,483 |
| C7 | 10 | 0 | 78,710 | 10,138 | 3,206 |
| C8 | 10 | 0 | 82,581 | 10,252 | 3,242 |
| C9 | 10 | 0 | 80,968 | 10,557 | 3,339 |
| C10 | 10 | 0 | 77,742 | 14,680 | 4,642 |
| C11 | 10 | 0 | 138,710 | 30,117 | 9,524 |

| Source of variation | DF | SS | MS | F | P |
|---|---|---|---|---|---|
| Between groups | 11 | 26,022,074 | 2,365,643 | 9,732 | <0,001 |
| Residual | 108 | 26,251,367 | 243,068 | | |
| Total | 119 | 52,273,441 | | | |

The differences in the mean values among the treatment groups are greater than would be expected by chance; there is a statistically significant difference (P = <0,001).
Power of performed test with alpha = 0,050: 1,000
Multiple Comparisons versus Control Group (Dunnett's Method) :

| Comparison | Diff of mean | q' | P | P<0,050 |
|---|---|---|---|---|
| FS Tem v C10 | 29,393 | 4.216 | <0.001 | Yes |
| FS Tern v C7 | 27,157 | 3.895 | <0.001 | Yes |
| FS Tem v C6 | 23,642 | 3.391 | <0.001 | Yes |
| FS Tem v C9 | 22,684 | 3.253 | <0.001 | Yes |
| FS Tem v C8 | 20,128 | 2.887 | 0.001 | Yes |
| FS Tem v C11 | 16,933 | 2.429 | -- | No |
| FS Tem v C1 | 7,668 | 1.100 | -- | Do not test |
| FS Tem v C3 | 4,473 | 0.642 | -- | Do not test |
| FS Tem v C5 | 3,854 | 0.553 | -- | Do not test |
| FS Tem v C4 | 3,514 | 0.504 | -- | Do not test |
| FS Tem v C2 | 0.319 | 0.0458 | -- | Do Not test |

Viability of stretch marks fibroblasts was significantly decreased for concentrations of 0.005% to 0.5%. The increase in cell viability observed with 1% of active was due to interference between the invention and culture medium. All the concentrations tested up to until 0.001% could be used for the experiments.

### One Way Analysis of Variance

Data source: Data 1 in Moyenne_Stats_FVR_Huile Normality Test: Failed (P < 0,050)
Equal Variance Test: Failed (P < 0,050)

| Group name | N | Missing | Mean | SD | SEM |
|---|---|---|---|---|---|
| FVR | 10 | 0 | 100,000 | 14,934 | 4,723 |
| C1 | 10 | 0 | 94,516 | 10,666 | 3,373 |
| C2 | 10 | 1 | 102,186 | 18,794 | 6,265 |
| C3 | 10 | 0 | 103,548 | 24,273 | 7,676 |
| C4 | 10 | 0 | 99,032 | 20,277 | 6,412 |
| C5 | 10 | 0 | 98,442 | 12,949 | 4,095 |
| C6 | 10 | 0 | 75,484 | 11,013 | 3,483 |
| C7 | 10 | 0 | 78,710 | 10,138 | 3,206 |
| C8 | 10 | 0 | 82,581 | 10,252 | 3,242 |
| C9 | 10 | 0 | 80,968 | 10,557 | 3,339 |
| C10 | 10 | 0 | 77,742 | 14,680 | 4,642 |
| C11 | 10 | 0 | 138,710 | 30,117 | 9,524 |

| Source of variation | DF | SS | MS | F | P |
|---|---|---|---|---|---|
| Between groups | 11 | 33,721,651 | 3,065,605 | 10,777 | <0,001 |
| Residual | 107 | 30,437,875 | 284,466 | | |
| Total | 118 | 64,159,526 | | | |

The differences in the mean values among the treatment groups are greater than would be expected by chance; there is a statistically significant difference (P = <0,001).
Power of performed test with alpha = 0,050: 1,000
Multiple Comparisons versus Control Group (Dunnett's Method): Comparisons for factor:

| Comparison | Diff of mean | q' | P | P<0,050 |
|---|---|---|---|---|
| FS Tem v C11 | 38,710 | 5,132 | <0.002 | Yes |
| FS Tem v C6 | 24,516 | 3,250 | <0.001 | Yes |
| FS Tem v C10 | 22,258 | 2,951 | <0.003 | Yes |
| FS Tem v C7 | 21,290 | 2,823 | <0.002 | Yes |
| FS Tem v C9 | 19,032 | 2,523 | 0.004 | Yes |
| FS Tem v C8 | 17,419 | 2,309 | 0.007 | Yes |
| FS Tem v C1 | 5,484 | 0.727 | -- | Do not test |
| FS Tem v C3 | 3,548 | 0.47 | -- | Do not test |
| FS Tem v C2 | 2,186 | 0.282 | -- | Do not test |
| FS Tem v C5 | 1,558 | 0.207 | - | Do not test |
| FS Tem v C4 | 968 | 0.128 | -- | Do Not test |

### PRIOR ART

### COMMON GENERAL KNOWLEDGE

Any proposed prior art is required to come within the common general knowledge before they can be considered as valid with which to challenge inventiveness.
1. Firstly, it would have to be explained how any study would be common general knowledge when, according to the PubMed database, it would be only one of millions of studies. The studies concerning the subject would amount to several million pages worth. So to read these studies would have taken around 10 to 20 years.
2. Section 2.8.1 of Case Law of the Boards of Appeal states that determining what constitutes common general knowledge plays an important role in the assessment of inventive step. Common general knowledge is defined as "knowledge that an experienced person in the field in question is expected to have, or at least to be aware of, to the extent that he knows he could look it up in a book if he needed it".
3. Section 2.8.2 of Case Law of the Boards of Appeal, going back as far as T206/83 (OJ 1987, 5) it was held that information which could only be obtained after a comprehensive search was not to be regarded as common general knowledge (see also T654/90, T924/03).
4. Section 2.8.2 of Case Law of the Boards of Appeal also states that according to T 412/09 patent specifications may only be considered to be common general knowledge when a series of patent specifications provides a consistent picture that a particular technical procedure was generally known.
5. Section 2.8.2 of Case Law of the Boards of Appeal, states that common general knowledge does not normally include patent literature and scientific articles (T206/83, T171/84, T307/11, T1641/11, T571/12, T1000/12).
6. Section 2.8.3 of Case Law of the Boards of Appeal also states that the board's view was that the content of specialist journals does not belong to the common general knowledge of the average skilled person because it was not normally part of that person's active knowledge either and had to be acquired through a comprehensive search.
7. Section 2.8.5 additionally states that "Where an assertion that something is common general knowledge is challenged, the person making the assertion must provide proof that the subject-matter in question is in fact common general knowledge (T 438/97, T 329/04, T 941/04, T 690/06)." Merely claiming it does, and ignoring all of the requirements concerning it, does not make it common general knowledge.

### INVENTIVENESS

In order to challenge the inventiveness of an invention, any potential prior art must first fulfill the following requirements of Case Law of the Boards of Appeal of the European Patent Office that are of particular relevance to the present application.
1. Case Law of the Boards of Appeal of the European Patent Office (Sixth Edition, July 2010) I. Patentability, D. Inventive Step, 3. Closest prior art, 3.1. Determination of closest prior art in general, and 3.2. Same purpose or effect states that: "The boards have repeatedly pointed out that the closest prior art for assessing inventive step is normally a prior art document disclosing subject-matter conceived for the same purpose. The first consideration is that it must be directed to the same purpose or effect as the invention. Otherwise, it cannot lead the skilled person in an obvious way to the claimed invention." It must also require "the minimum of structural and functional modifications."
2. Case Law of the Boards of Appeal of the European Patent Office (Sixth Edition, July 2010) I. Patentability, D. Inventive Step, 5. "Could-would approach" and ex post facto analysis states that : In T970/00 the board stated that any ex post facto analysis, and in particular any conclusion going beyond what the skilled person would have objectively inferred from the prior art, without the benefit of hindsight knowledge of the invention, is of necessity at variance with a proper application of the problem-solution approach. It is the boards' established case law that the question is not whether the skilled person could have carried out the invention, but whether he would have done so in the hope of solving the underlying technical problem or in the expectation of some improvement or advantage - the so-called "could-would approach" (T 2/83, OJ 1984, 265; T 90/84, T 7/86, OJ 1988, 381; T 200/94, T 885/97). It has been held that once an invention existed, it could often be shown that the skilled person could have made it by combining different elements in the prior art, but such arguments had to be disregarded as the product of ex post facto analysis (T 564/89).
3. According to "Case Law of the Boards of Appeal of the European Patent Office (Sixth Edition, July 2010) I. Patentability, D. Inventive Step, 5. Could-would Approach" : In Case T 0970/00 Accordingly, the determination of the technical contribution achieved by the invention over the closest state of the art requires an objective and technically meaningful and consistent comparison of the claimed combination of structural and functional features. Any attempt to interpret the disclosure of the closest prior art so as to distort or misrepresent, based on hindsight knowledge of the invention, the proper technical teaching of the disclosure in such a way that it artificially meets specific features recited in the claim under consideration must therefore fail.
4. Case Law of the Boards of Appeal of the European Patent Office (Sixth Edition, July 2010) I. Patentability, D. Inventive Step, 5. "Could-would approach" and ex post facto analysis also states that : "It is the boards' established case law that the question is not whether the skilled person could have carried out the invention, but whether he would have done so in the hope of solving the underlying technical problem or in the expectation of some improvement or advantage - the so-called "could-would approach" (T 2/83, OJ 1984, 265; T 90/84, T 7/86, OJ 1988, 381; T 200/94, T 885/97).

The potential prior art, besides not coming within the common general knowledge, fails to fulfill all of the legal requirements of the Case Law of the Boards of Appeal of the European Patent Office in order to be considered valid prior art with which to challenge the inventiveness of the present invention.

### INDUSTRIAL APPLICABILITY

This invention concerns a cosmetic or pharmaceutical preparation whose purpose is to have a preventive effect on the formation of stretch marks through a reduction in the contractile forces developed by fibroblasts during early stretch mark development. The active formulation consists of Avocado Oil, Olive Oil, Coconut Oil, Rose Hip Oil, Wheatgerm Oil, Macadamia Nut Oil, Shea Butter, Natural Mixed Tocopherols, Argan Oil, Calendula Oil, Natural Orange Oil, Lavender Oil, and Vitamin A Palmitate. The preparation does not, unlike other preparations, include any paraffin oils, petroleum derivatives, additives or waxes.

## Claims

1. A cosmetic or pharmaceutical preparation intended for application to skin in order to prevent the early formation of stretch marks with alteration of the contractile forces developed by Fibroblasts, that comprises avocado oil, olive oil and coconut oil, in which avocado oil and olive oil form the majority of the composition according to weight.

2. A cosmetic or pharmaceutical preparation intended for application to skin with alteration of the contractile forces developed by fibroblasts in order to prevent the early formation of stretch marks that in which avocado oil, olive oil and coconut oil form more than two thirds of the composition according to weight.

3. A composition according to claim 2 that also includes wheatgerm oil and macadamia nut oil.

4. A composition according to claim 3 that also includes shea butter, argan oil, Rose Hip oil and calendula oil.

5. A composition according to claim 4 that also includes natural orange oil and lavender oil.

6. A composition according to claim 5 that also includes : natural mixed tocopherols, and vitamin A palmitate.

7. A cosmetic or pharmaceutical preparation intended for application to skin in order to prevent the early formation of stretch marks with alteration of the contractile forces developed by fibroblasts that comprises avocado oil, olive oil, coconut oil, wheatgerm oil, macadamia nut oil, shea butter, argan oil, Rose Hip oil, calendula oil, orange oil, lavender oil, natural mixed tocopherols, and vitamin A palmitate.

8. A composition according to any one of the claims, in which paraffin oils, petroleum derivatives, additives or waxes are not included in the composition.

9. A composition according to any one of the claims in which the composition includes one or more carriers or excipients.

10. A composition according to any one of claims wherein the composition consists essentially of the components defined in that claim.
